(19) **Europäisches Patentamt**
**European Patent Office**
**Office européen des brevets**

(11) **EP 2 225 392 B1**

(12) **EUROPEAN PATENT SPECIFICATION**

(45) Date of publication and mention
of the grant of the patent:
**11.12.2013 Bulletin 2013/50**

(51) Int Cl.:
***C12Q 1/68*** (2006.01)

(21) Application number: **08864740.9**

(22) Date of filing: **19.12.2008**

(86) International application number:
**PCT/EP2008/068083**

(87) International publication number:
**WO 2009/080780 (02.07.2009 Gazette 2009/27)**

(54) **A METHOD OF DIAGNOSING A PROGRESSIVE DISEASE**

VERFAHREN ZUR DIAGNOSE EINER FORTSCHREITENDEN ERKRANKUNG

PROCÉDÉ DE DIAGNOSTIC D'UNE MALADIE ÉVOLUTIVE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.12.2007 EP 07450240**

(43) Date of publication of application:
**08.09.2010 Bulletin 2010/36**

(73) Proprietor: **Mayer, Gert**
**6020 Innsbruck (AT)**

(72) Inventors:
• **RUDNICKI, Michael**
**A-6170 Zirl (AT)**
• **EDER, Susanne**
**A-6091 Birgitz (AT)**
• **SCHRATZBERGER, Gabriele**
**A-6020 Innsbruck (AT)**
• **ENRICH, Julia**
**A-6170 Zirl (AT)**
• **PERCO, Paul**
**1080 Vienna (AT)**

(74) Representative: **Redl, Gerda**
**REDL Life Science Patent Attorneys**
**Tech Gate Vienna**
**Donau-City-Straße 1**
**1220 Vienna (AT)**

(56) References cited:
WO-A-02/090585        WO-A-2007/028636
JP-A- 2007 327 921

• **HUNLEY T E ET AL: "ANGIOTENSIN CONVERTING ENZYME GENE POLYMORPHISM: POTENTIAL SILENCERMOTIF AND IMPACT ON PROGRESSION IN IGA NEPHROPATHY" KIDNEY INTERNATIONAL, NEW YORK, NY, US, vol. 49, 1 January 1996 (1996-01-01), pages 571-577, XP002926340 ISSN: 0085-2538**
• **RUDNICKI M ET AL: "Gene expression profiles of human proximal tubular epithelial cells in proteinuric nephropathies" KIDNEY INTERNATIONAL, vol. 71, no. 4, February 2007 (2007-02), pages 325-335, XP002484287 ISSN: 0085-2538**
• **RUDNICKI MICHAEL ET AL: "Reliability of t7-based mRNA linear amplification validated by gene expression analysis of human kidney cells using cDNA microarrays." NEPHRON. EXPERIMENTAL NEPHROLOGY 2004, vol. 97, no. 3, 2004, pages e86-e95, XP008093243 ISSN: 1660-2129 cited in the application**

Remarks:
The file contains technical information submitted after the application was filed and not included in this specification

**Description**

The present invention relates to a method for predicting the progression of chronic kidney disease by measuring a panel of biomarkers.

The increasing prevalence of patients on renal replacement therapy has become a major challenge for healthcare systems. Frequently, end stage renal disease (ESRD) is the terminal phase of a chronic process. A better understanding of the pathophysiology of progressive kidney disease could lead to the development of new treatment options which might be able to stabilize renal function and reduce the incidence of ESRD. In order to use new but also the already available drugs even more efficiently, it is also highly desirable to identify patients with an adverse renal prognosis in the early phases of the disease as not all subjects show a relentlessly progressive decline in renal function. In this context the magnitude of proteinuria has been suggested to be a useful risk marker, even though, on an individual basis, the discriminatory power is questionable. Other biomarkers such as apolipoprotein A-IV (APOA4), adiponectin (ADIPOQ), or fibroblast growth factor 23 (FGF23) have recently been proposed as alternative parameters to predict the course of disease.

WO 2007/028636 describes a method for predicting the progression of chronic kidney disease by measuring apolipoprotein A-IV.

Rudnicki et al (Nephron Exp Nephrol 2004; 97:e86-e95) describe gene expression analysis of a human kidney cell line using cDNA microarrays, and a correlation between microarray  and qRT-PCR results. Rudnicki et al (Kidney International 2007, 71, 325-335) disclose the gene expression profiles of human proximal tubular epithelial cells in proteinuric nephropathies. 168 different genes have been characterized.

It is a goal of the present invention to provide suitable markers to identify patients at risk of renal disease progression.

The object is solved by the method according to the invention, which provides for the determination of the risk of progressive renal disease in a patient, by measuring a parameter related to a marker selected from the group of IL1RN, ISG15, LIFR, C6, IL32 and any combination thereof, in a sample of said patient, preferably a combination that contains at least one of IL1RN, IL32 and LIFR.

The method according to the invention preferably refers to the combination of two or three markers.

According to the inventive method the progressive disease is preferably indicated, if the amount of said marker is increased, e.g. by at least 1.1 times the reference value of subjects not suffering from the progressive disease.

Preferably the sample is a blood, serum, plasma, urine sample or a tissue, such as a kidney biopsy sample.

As a parameter, the amount of the marker or a factor related thereto may be measured. For instance the expression of said marker is preferably determined, such as by nucleic acid and/or protein expression of said marker.

The method according to the invention in particular refers to the determination of said parameter by microarray hybridization with specific probes, or by PCR.

It is preferred that the method according to the invention further employs the determination of a senescence parameter.

By the method according to the invention a renal disease is determined, in particular selected from IgA nephropathy, non IgA mesangioproliferative glomerulonephritis, membranoproliferative glomerulonephritis, any postinfectious glomerulonephritis, focal-segmental glomerulosclerosis, minimal change disease, membranous nephropathy, lupus nephritis of any kind, vasculitides with renal involvement of any kind, any other systemic disease leading to renal disease including but not being limited to diabetes mellitus, hypertension or amyloidosis, any hereditary renal disease, any interstitial nephritis and renal transplant failure.

The inventive method is preferably used for the prognosis of kidney failure, as well as for the diagnosis of renal disease in a patient at risk of disease progression.

According to the invention there is further provided a set of reagents, which set has specificities for determining two to five markers selected from the group consisting of IL1RN, ISG15, LIFR, C6 and IL32.

The set according to the invention preferably contains antibodies or antibody fragments, or nucleotide sequence specific oligonucleotides as reagents. In a preferred embodiment the reagents are labelled.

Therefore, the present invention provides a method of determining progressive disease, e.g. the risk of disease conditions associated with end-stage renal failure. A method for diagnosing a progressive disease and/or assessing long term prognosis of a disease is particularly important to qualify high risk patients early on, even before the diagnosis of a chronic disease.

It has been surprisingly found out that a marker selected from IL1RN, ISG15, LIFR, C6, IL32 or any combination thereof is determinative of high risk patients. For the inventive method one of these markers or associated parameters can be detected, or a combination of any two, three, four, or five of these markers. Associated parameters relate to genotypic or phenotypic analytes, which relate to the specific markers with a high correlation.

The inventive markers are described as follows:

1. C6 - Complement component 6 (UniGene: Hs.481992, GeneID: 729, GenBank: N59396): C6 is a component of complement cascade. It is part of the membrane attack complex which can insert into the cell membrane and cause cell to lyse. People with C6 deficiency are prone to bacterial infection.

2. IL32 - Interleukin 32 (UniGene: Hs.943, GeneID: 9235, GenBank: AA458965): This gene encodes a member of the cytokine family. The protein contains a tyrosine sulfation site, 3 potential N-myristoylation sites, multiple putative phosphorylation sites, and an RGD cell-attachment sequence. Expression of this protein is increased after the activation of T-cells by mitogens or the activation of NK cells by IL-2. This protein induces the production of TNFalpha from macrophage cells. Alternate transcriptional splice variants, encoding different isoforms, have been characterized.

3. ISG15 - ISG15 ubiquitin-like modifier (UniGene: Hs.458485, GeneID: 9636, GenBank: AA406019 / AA120862).

4. IL1RN - interleukin 1 receptor antagonist (UniGene: Hs.81134, GeneID: 3557, GenBank: T71181): The protein encoded by this gene is a member of the interleukin 1 cytokine family. This protein inhibits the activities of interleukin 1, alpha (IL1A) and interleukin 1, beta (IL1B), and modulates a variety of interleukin 1 related immune and inflammatory responses. This gene and five other closely related cytokine genes form a gene cluster spanning approximately 400 kb on chromosome 2. A polymorphism of this gene is reported to be associated with increased risk of osteoporotic fractures and gastric cancer. Four alternatively spliced transcript variants encoding distinct isoforms have been reported.

5. LIFR - leukemia inhibitory factor receptor alpha (UniGene: Hs.133421, GeneID: 3977, GenBank: AI820550): The leukemia inhibitory factor is a polyfunctional cytokine that affects the differentiation, survival, and proliferation of a wide variety of cells in the adult and the embryo. LIF action appears to be mediated through a high-affinity receptor complex composed of a low-affinity LIF binding chain (LIF receptor) and a high-affinity converter subunit, gp130. Both LIFR and gp130 are members of a family of cytokine receptors that includes components of the receptors for the majority of hematopoietic cytokines and for cytokines that affect other systems, including the ciliary neurotrophic factor, growth hormone and prolactin.

It was found that other renal disease parameters, such as p16 (INK4A/ARF, CDKN2A) or senescence parameters could be preferably combined with any one of the foregoing markers to improve the significance of the determination. Senescence parameters are preferably selected from the group consisting of chronological age, telomere length and CDKN1A. Other senescence parameters commonly used to determine a correlation with chronological age may be employed as well, such as those, which are either regulators of p53, associated with DNA repair, cell cycle control, telomere binding and cell surface remodelling. Exemplary senescence associated genes are selected from the group consisting of Sirtiuns 1-8, XRCC5, G22P1, hPOT 1, Collagenase, TANK 1,2, TRF 1,2 and WRN.

As a read out, the amount of parameters in a sample may be determined and correlated to the risk of said patients, which can be low, medium or high, or else prediction rules established in order to discriminate between the binary outcome stable or progressive disease. For example, the ability of a prediction rule was assessed by calculating the area under the ROC curve (AUC) using the Sommer's D statistic. The relation between the area under the ROC and Sommer's D is the following:

$$AUC = (1 + Sommer's\ D)/2.$$

It is preferred to employ a marker according to the invention either as single predictor of progression with an AUC value of at least 0.5, preferably at least 0.6, more preferred 0.7, 0.8 or even at least 0.9. Preferred marker combinations reach AUC values of at least 0.6, preferably at least 0.7, 0.8 or even at least 0.9, up to 1.0.

With reference to a healthy patient or a stable disease patient, the preferred method according to the invention qualifies a significant risk when an increase of single parameters by at least 10%, preferably at least 20%, more preferred at least 30%, more preferably at least 40%, more preferably at least 50% is determined.

The high risk progressive nature of the disease is preferably indicated, if the amount of a marker or the combination of markers is increased at least 1.5 times the reference value of subjects not suffering from the progressive disease, preferably being healthy subjects or subjects suffering from a chronic non-progressive disease.

In special embodiments the amount of IL1RN is at least 1.5, preferably at least 1.6, at least 1.8, at least 2, at least 3, at least 4, at least 5, at least 6, or at least 8 times the reference value, in particular as determined by PCR with either PPIA or GAPDH as endogenous controls or as determined by microarray analysis.

In special embodiments the amount of ISG15 is at least 1.5, preferably at least 1.6, at least 1.8, at least 2, at least 3, at least 4, at least 5 or at least 6 times the reference value, in particular as determined by PCR with either PPIA or GAPDH as endogenous controls or as determined by microarray analysis.

In special embodiments the amount of LIFR is at least 1.5, preferably at least 1.6, at least 1.8, at least 2 or at least 3 times the reference value, in particular as determined by PCR with either PPIA or GAPDH as endogenous controls or as determined by microarray analysis.

In special embodiments the amount of C6 is at least 1.5, preferably at least 1.6, at least 1.7, at least 1.8 or at least 2 times the reference value, in particular as determined by PCR with either PPIA or GAPDH as endogenous controls or as determined by microarray analysis.

In special embodiments the amount of IL32 is at least 1.5, preferably at least 1.6, at least 1.7, at least 1.8 or at least 2 times the reference value, in particular as determined by PCR with either PPIA or GAPDH as endogenous controls or as determined by microarray analysis.

If more than one marker is detected, the comparison is made to each single reference value for each marker in the non-progressive disease or healthy reference itself.

The inventive method can distinguish if a chronic disease is stable, i.e. the symptoms do not significantly increase over a period of about at least or up to four, six, eight, ten months, one, two or three years after the sample was obtained, or is a progressive disease, i.e. the condition of the subject will increasingly suffer, e.g. over the same time span.

Patients at risk of a renal progressive disease have an increased risk of gradual worsening of renal disease.

The National Kidney Foundation's Kidney Disease Outcomes Quality Initiative (NKF KDOQI) classified chronic kidney disease (CKD) into five stages with stage five indicating terminal kidney failure. Stage 1 patients have kidney damage with normal glomerular filtration rate (GFR) values above 90ml/min/1.73m2. Patients in stage two have slightly decreased GFR values between 60 and 89 ml/min/1.73m2. Stage three patients have moderately decreased GFR values between 30 and 59 ml/min/1.73m2. Patients in stage four experience severely decreased GFR values between 15-29 ml/min/1.73m2. Kidney failure, also defined as end-stage renal disease, is reached in stage five when patients have GFR values lower than 15 ml/min/1.73m2. End-stage renal disease is followed by renal replacement therapy with the treatment options dialysis or organ transplantation.

If the risk of end-stage renal failure is high, the disease stages will be passed very quickly, which would result in the need for kidney dialysis and transplantation. To delay the terminal phase of renal disease a patient which was diagnosed as having an increased risk of disease progression would receive the appropriate medication early on employing aggressive treatment regimens.

The risk of a patient to suffer from kidney or renal disease progression may be diagnosed at an early stage of disease, even before a chronic disease has been diagnosed. On the other hand a prognosis is provided, which would quantify the fast progression of the disease in a patient already suffering from chronic renal disease.

Thus, the inventive method can include the step of obtaining the sample from a patient potentially suffering from a progressive renal disease, where a chronic renal disease may already have been diagnosed or not.

The inventive markers can be detected in any sample of a subject comprising said markers e.g. either as mRNA or expressed protein. Preferably the DNA is not used as parameter to determine the marker. The comparison with the reference value should be of the same sample type. In particular, the sample can be tissue, e.g. of a biopsy, blood, serum, plasma or a urine sample.

In preferred embodiments, determining the amount of the marker or any combination thereof comprises determining the expression of the marker(s), preferably by determining the mRNA concentration of the marker(s). To this extend, mRNA of the sample can be isolated, if necessary after adequate sample preparation steps, e.g. tissue homogenisation, and hybridized with marker specific probes, in particular on a microarray platform with or without amplification, or primers for PCR-based detection methods, e.g. PCR extension labelling with probes specific for a portion of the marker mRNA. In preferred embodiments the marker(s) or a combination thereof is (are) determined by microarray hybridization with IL1RN, ISG15, LIFR, C6, IL32 specific probes, or by PCR.

In further embodiments the amount of a marker or any combination thereof is determined by the protein concentration of the marker(s), e.g. with marker specific antibodies or binding partners. The binding event can, e.g. be detected by competitive or non-competitive methods, including the use of labelled marker specific moieties, e.g. antibodies, or labelled competitive moieties, including a labelled marker standard, which compete with marker proteins for the binding event.

The condition which can be detected with the inventive methods is in particular a progressive renal disease, which can e.g. be determined by using a kidney biopsy sample, such as wedge or needle sample, or else from tubular cells, and also by detecting the markers in serum, blood, plasma and urine by comparing reference values of non-progressive renal disease values or from healthy subjects.

The subject can, e.g., be any mammal, in particular a human, but also selected from vertebrate animals, such as mouse, rat, hamster, cat, dog, horse, cow, pig, etc.

In a further aspect the present invention provides a set that contains or consists of at least two different reagents or marker specific moieties, to determine two, three, four, preferably not more than five markers, wherein the markers are selected from IL1RN, ISG15, LIFR, C6 or IL32. Besides, further markes may be determined in the same sample for a different purpose. The set preferably contains reagents that provide for the determination of only a few markers as necessary to determine the risk of disease progression, e.g. of a maximum of five of the markers, most preferably only the two or three best suitable markers. Anyone skilled in the art will quickly find out, which of the parameters related to one or more of the five markers according to the invention are most determinative in various different samples and with different reagents.

Marker specific moieties used as reagents according to the invention are substances which can bind to or detect at least one of the markers for a detection method described above and are in particular marker protein specific antibodies or antibody fragments, such as Fab, F(ab)2, F(ab)', Fv, scFv, or single chain antibodies. The marker specific moieties can also be selected from marker nucleotide sequence specific oligonucleotides, which specifically bind to a portion of the  marker sequences (e.g. mRNA or cDNA) or are complementary to such a portion in the sense or complementary anti-sense (cDNA complementary strand) orientation. For easy detection the moieties are preferably labelled, such as by optical, including fluorescence, and radioactive labels.

The present invention is further illustrated by the following figures and examples without being limited thereto.

Figures:

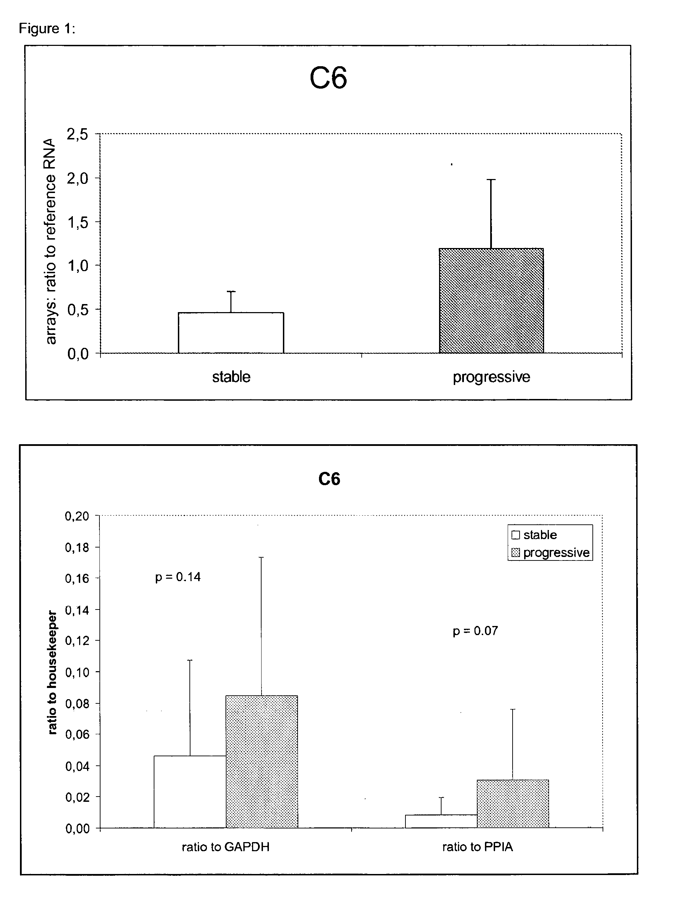

Fig. 1: Results for C6: Arrays: 14 SD vs 7 PD (fold-change for N59396: 2.27), rtPCR: 11 SD vs 9 PD (fold-change to PPIA: 3.88, fold-change to GAPDH: 1.85)

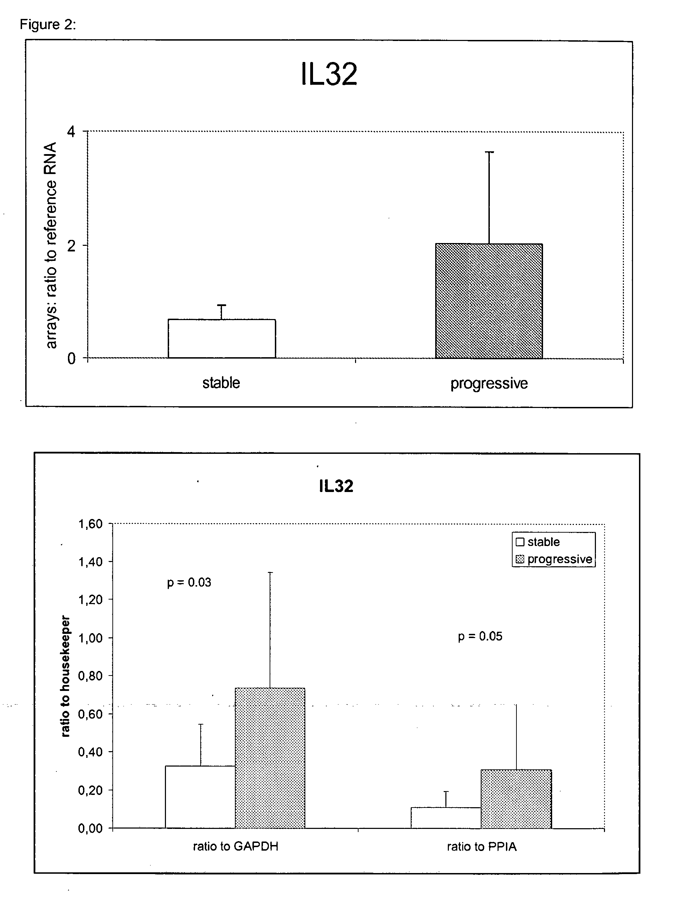

Fig. 2: Results for IL32: Arrays: 14 SD vs 7 PD (fold-change for AA458965: 2.78), rtPCR: 11 SD vs 9 PD (fold-change to PPIA: 2.86, fold-change to GAPDH: 2.25)

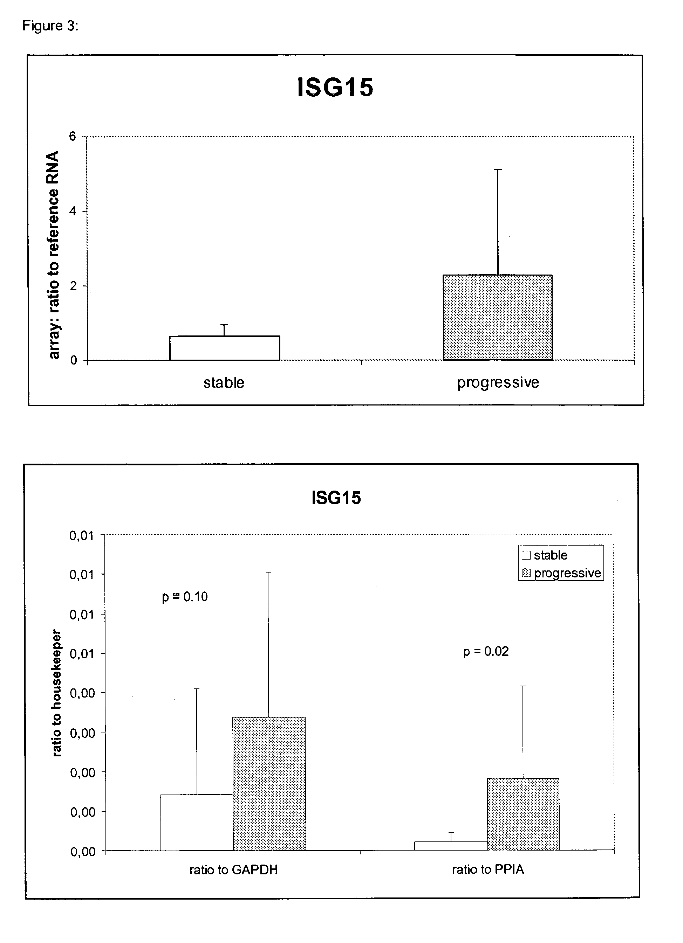

Fig. 3: Results for ISG15: Arrays: 14 SD vs 7 PD (fold-change for AA406019/AA120862: 2.79 and 2.19), rtPCR: 11 SD vs 10 PD (fold-change to PPIA: 8.27, fold-change to GAPDH: 2.40)

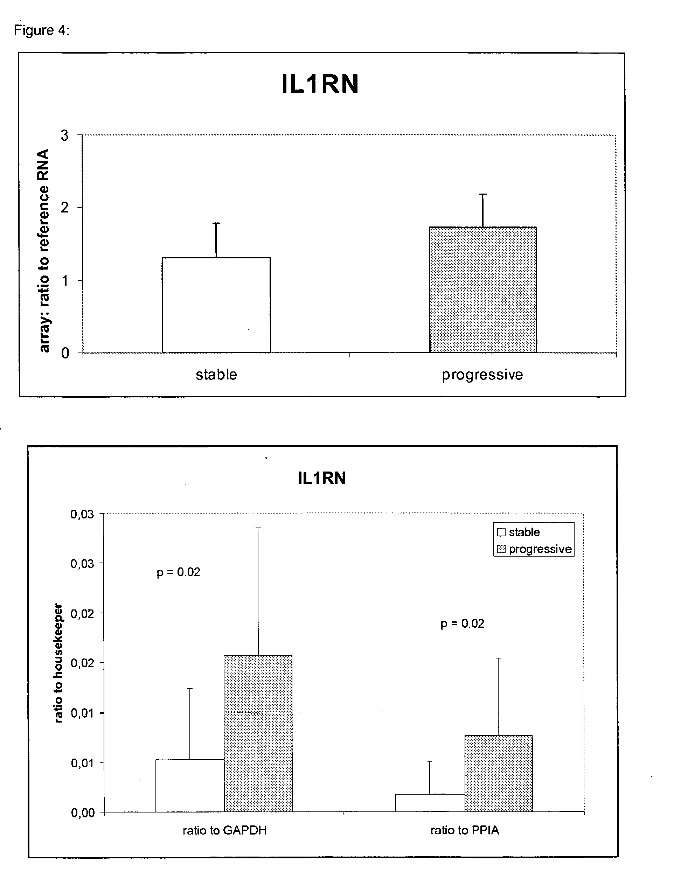

Fig. 4: Results for IL1RN: Arrays: 14 SD vs 7 PD (fold-change for T71181: 1.69), rtPCR: 11 SD vs 10 PD (fold-change to PPIA: 4.40, fold-change to GAPDH: 2.98)

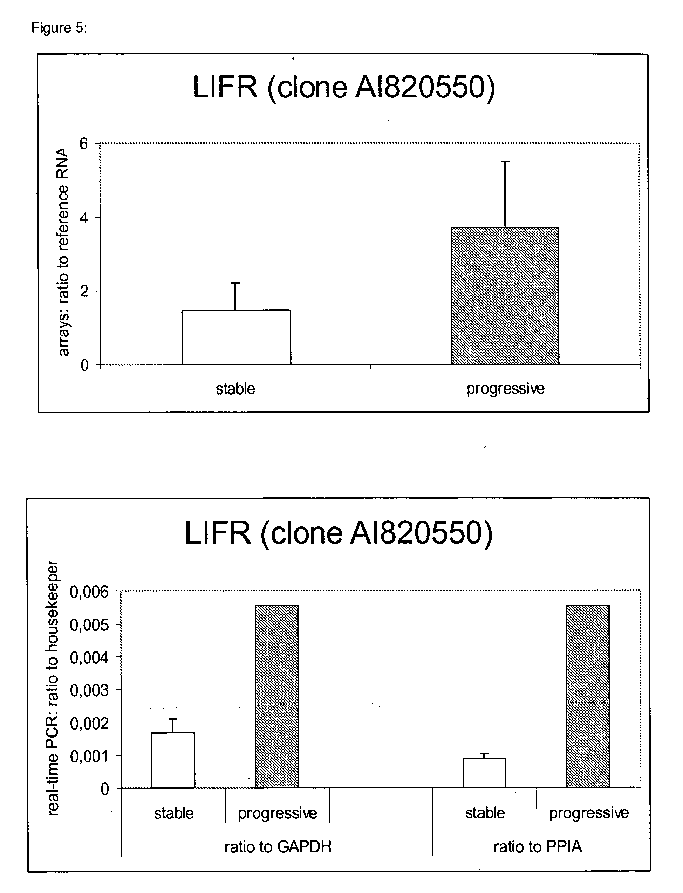

Fig. 5: Results for LIFR: Arrays: 14 SD vs 7 PD (fold-change for AI820550: 2.46), rtPCR: 3 SD vs 3 PD (fold-change to PPIA: 2.64, fold-change to GAPDH: 3.47)

The invention is further described by the following examples.

Examples:

Example 1: Patient Samples

34 kidney biopsies obtained from patients with proteinuric renal diseases during their routine diagnostic workup were used. The histological diagnoses were IgA nephropathy (IGAN) in 14, focal-segmental glomerulosclerosis (FSGS) in 5, minimal change disease (MCD) in 3, lupus nephritis WHO class IV (SLE) in 4 and anti-GBM rapidly progressive glomerulonephritis (RPGN) in 2 patients. One subject each suffered from membranous nephritis (MN), membranoproliferative nephritis (MPGN), p-ANCA positive microscopic polyangiitis (MPA), vascular nephropathy (VNP), MPGN with purpura Schoenlein-Henoch and diabetic nephropathy (DN). Microarray-based gene expression profiling was performed in 21 of these patients' samples, while real-time PCR validation experiments were performed in 20 with 7 samples being analysed by both microarray and real-time PCR.

Based on the course of excretory kidney function during a 2 to 46 month follow-up period after biopsy, this cohort was split into subjects with "stable disease (SD)" and "progressive disease (PD)". Patients were defined as progressive when they experienced a persistent rise of at least 50% in serum creatinine during follow-up, or when they reached end-stage renal disease (n=14). All other patients were defined as stable (n=20). Light microscopy of the biopsies was performed using hematoxylin/eosin and periodic-acid-Schiff (PAS) or Pearse-stained sections. Scoring of tubulointerstitial fibrosis was performed by an independent pathologist following a semiquantitative grading system: 0 no fibrosis; 1: < 10%; 2: 10 - 25; 3: 25 - 50; 4: 50 - 75; 5: > 75%. Material used for microarray studies was obtained from cryo-cut sections.

Example 2: RNA isolation and microarray hybridization

Processing of frozen sections, isolation of proximal tubular cells, isolation of total RNA, quality control by RT-PCR and T-7 based linear amplification of RNA were performed as recently described by Rudnicki et al. (Kidney Int 2007; 71: 325-35). The linearity and reproducibility of two rounds of RNA amplification for microarray experiments using RiboAmp™ RNA amplification kit (Arcturus, Mountain View, CA, USA) has also been evaluated. Two rounds amplified Universal Human Reference RNA™ (Stratagene, La Jolla, CA, USA) was used as reference RNA in all experiments. Using the CyScribe cDNA Post Labelling Kit (formerly Amersham Biosciences, now GE healthcare life sciences, Piscataway, NJ, USA), 1 $\mu$g of amplified sample RNA (Cy5, red) and 1$\mu$g of amplified reference RNA (Cy3 green) were labeled and cohybridized to the arrays as described by Rudnicki et al. (Nephon Exp Nephrol 2004; 97: e86-95). cDNA microarrays were obtained from the Stanford Functional Genomics Facility (www.microarray.org/sfgf/). The arrays contained 41792 spots, representing 30325 genes assigned to a UniGene cluster and 11467 ESTs. All samples were processed in technical duplicates. Arrays were scanned using a GenePix 4000B microarray scanner and the images were analyzed with the GenePix Pro 4.0 software (Axon Instruments, Union City, CA).

Example 3: Statistical analysis and selection of putative biomarkers

Signals showing intensity signal over background values < 2.5 in either channel were excluded and the analyses were focused on genes with valid data in at least 80% of processed samples, leaving 19921 cDNA clones in the analysis dataset. Gene expression values of technical array replicates were averaged. A two-sample t-test ($p < 0.05$) and the two-fold-change criterion were used to identify differentially expressed genes (DEGs) when comparing both patient cohorts.
The subcellular location of DEGs was determined using data stored in the SwissProt database, and secreted proteins were identified. The secreted DEGs showing the highest fold-change values were selected for validation via real-time PCR experiments.

Example 4: Validation via real-time PCR

The TaqMan™ PreAmp Master Mix (Ambion, Austin, TX, USA) together with the respective TaqMan™ probes (vide infra) was used for 300-400fold amplification of the original RNA as the laser-capture microdissection of proximal tubular cells from frozen sections yields about 1 ng of total RNA per sample. In all real-time PCR experiments GAPDH (Hs99999905_m1) and PPIA (cyclophilin A; Hs99999904_m1) were used as endogenous controls. Based on the ratios to housekeeper genes (i.e. 2 exp delta ct) a fold-change between stable and progressive disease patients was calculated and compared to the fold-change values obtained by microarray analysis.

Example 5: Microarray analysis

Of the 113 DEGs upregulated in the progressive disease samples six had protein isoforms which were secreted according to information stored in the SwissProt database. These genes are the complement component 6 (C6), interleukin 32 (IL32), ISG15 ubiquitin-like modifier (ISG15), the leukemia inhibitory factor receptor alpha (LIFR), transferring (TF), and TIMP metallopeptidase inhibitor 1 (TIMP1). In addition, the interleukin 1 receptor antagonist was included due to its very low p-value of < 0.0001.

| GeneName | Gene Symbol | p-value | Fold-change (array) |
| --- | --- | --- | --- |
| TIMP metallopeptidase inhibitor 1 | TIMP1 | 0.00010 | 4.24 |
| Transferrin | TF | 0.00815 | 3.79 |
| ISG15 ubiquitin-like modifier | ISG15 | 0.00575 | 2.79 |
| Interleukin 32 | IL32 | 0.01162 | 2.78 |
| Complement component 6 | C6 | 0.00036 | 2.27 |
| Leukemia inhibitory factor receptor alpha | LIFR | 0.01169 | 2.02 |
| Interleukin 1 receptor antagonist | IL1RN | 0.00005 | 1.69 |

Example 6: Validation via real-time PCR

The upregulation of five out of the seven selected biomarkers could be validated in rtPCR experiments. Two markers showed a downregulation, namely TF and TIMP1, i.e. these proteins were not confirmed in rtPCR.

| GeneName | Gene-Symbol | p-value | Fold-change array | Fold-change (rtPCR to PPIA) | Fold-change (rtPCR to GAPDH) |
|---|---|---|---|---|---|
| Interleukin 1 receptor antagonist | IL1RN | 0.00005 | 1.69 | 4.40 | 2.98 |
| ISG15 ubiquitin-like modifier | ISG15 | 0.00575 | 2.79 | 8.27 | 2.40 |
| Leukemia inhibitory factor receptor alpha | LIFR | 0.01169 | 2.02 | 2.64 | 3.47 |
| Complement component 6 | C6 | 0.00036 | 2.27 | 3.88 | 1.85 |
| Interleukin 32 | IL32 | 0.01162 | 2.78 | 2.86 | 2.25 |
| TIMP metallopeptidase inhibitor 1 | TIMP1 | 0.00010 | 4.24 | 1.11 | -1.35 |
| Transferrin | TF | 0.00815 | 3.79 | 1.28 | -2.76 |

Example 7 - Discriminatory power of single markers and marker models based on microarray data

Based on gene expression data of the five markers under study we established prediction rules in order to discriminate between the binary outcome stable or progressive disease. We assessed the ability of the prediction rule by calculating the area under the ROC curve (AUC) using the Sommer's D statistic. The relation between the area under the ROC and Sommer's D is AUC = (1+Sommer's D)/2. AUC values of 1 indicate complete discrimination of the two groups based on the marker values, whereas values of 0.5 indicate random assignment.

In this study the best single predictor of progression with an AUC value of 0.969 is IL1RN, followed by C6 (AUC = 0.958) and IL32 (AUC = 0.929). Preferred marker combinations reaching all AUC values of 1 are for example IL32 and IL1RN, IL32 and LIFR, C6 and IL1RN, C6 and LIFR, as well as IL1RN and ISG15. A complete listing of AUC values of the respective markers and marker combinations based on gene expression data is given in the table below.

| Marker (combination) | AUC |
|---|---|
| IL32 | 0.929 |
| C6 | 0.958 |
| ISG15 | 0.814 |
| IL1RN | 0.969 |
| LIFR | 0.827 |
| IL32 + C6 | 0.972 |
| IL32 + ISG15 | 0.917 |
| IL32 + IL1RN | 1 |
| IL32 + LIFR | 1 |
| C6 + ISG15 | 0.938 |
| C6 + IL1RN | 1 |
| C6 + LIFR | 1 |

(continued)

| Marker (combination) | AUC |
|---|---|
| ISG15 + IL1RN | 1 |
| ISG15 + LIFR | 0.886 |
| IL1RN + LIFR | 0.980 |
| IL32 + C6 + ISG15 | 0.958 |
| IL32 + C6 + IL1RN | 1 |
| IL32 + C6 + LIFR | 1 |
| IL32 + ISG15 + IL1RN | 1 |
| IL32 + ISG15 + LIFR | 1 |
| IL32 + IL1RN + LIFR | 1 |
| C6 + ISG15 + IL1RN | 1 |
| C6 + ISG15 + LIFR | 1 |
| C6 + IL1RN + LIFR | 1 |
| ISG15 + IL1RN + LIFR | 1 |
| IL32 + C6 + ISG15 + IL1RN | 1 |
| IL32 + C6 + ISG15 + LIFR | 1 |
| IL32 + C6 + IL1RN + LIFR | 1 |
| IL32 + ISG15 + IL1RN + LIFR | 1 |
| C6 + ISG15 + IL1RN + LIFR | 1 |
| IL32 + C6 + ISG15 + IL1RN + LIFR | 1 |

Example 8 - Validation via ELISA on protein level

Protein levels were determined in patient plasma in a prospective dataset consisting of patients with a stable course of disease [n=20], patients with a progressive course of disease [n=16], as well as reference samples of healthy volunteers [n=30] for the two markers IL1RN and LIFR.

Quantitation of human LIFR in plasma of patients/healthy controls was carried out by utilizing the Biosource® Human sLIF-R/gp190 ELISA Kit (Invitrogen, California, U.S.). Samples were used undiluted, standard dilutions ranged from 5 to 0.08ng/ml (100μl/well, arranged in triplicates). Sandwich ELISA procedure was conducted as described by the Protocol Booklet. Adsorbance was read on a spectrophotometer using 450nm as the wavelength.

Quantikine® Human IL-1ra/IL-1F3 Immonoassay (R&D Systems, Minneapolis, U.S.) was performed according to the manufacturer's protocol in order to measure IL1RN protein concentrations. Plasma from patients suffering from chronic kidney disease and healthy controls was diluted 1:20 using the Calibrator Dilutent provided by the kit. 100ul of standards (ranging from 2000 to 31.2pg/ml) and samples were pipetted into the appropriate wells (determination in triplicates). OD values were measured at 450nm with wavelength correction at 540nm.

The sandwich ELISA procedure for the quantitation of C6 was conducted as described by Würzner et al., Clin. Exp. Immunol. (1991), 83, 430-437. In brief, monoclonal Ab directed against C6 (provided by R. Würzner, Department of Hygiene, Microbiology and Social Medicine, Innsbruck Medical University, Austria) was adsorbed to microplate wells (1.5ug/well), followed by incubation of human plasma (stable and progressive patients as well as healthy volunteers) at a 1:1000 dilution (100μl each). Biotinylated polyclonal goat anti-C6 IgG was then added to each well (2.5μg/ml) followed by addition of HRP-labeled streptavidin (dilution 1:500). Finally, ABTS was introduced and the absorption at 410nm was recorded. Data were evaluated by comparison to a standard ranging from 900 to 14pg/ml.

Mean C6 concentrations in the two patient groups, stable and progressive, as well as in the group of healthy volunteers were 130 ng/μl, 111 ng/μl, and 82 ng/μl respectively.

Mean LIFR concentrations in the two patient groups, stable and progressive, as well as in the group of healthy volunteers were 5.2 ng/ml, 6 ng/ml, and 5.2 ng/ml  respectively.

Mean IL1RN concentrations in the two patient groups, stable and progressive, as well as in the group of healthy volunteers were 31 pg/ml, 40 pg/ml, and 19 pg/ml respectively.

In a similar way as in example 7 the AUC values were determined thus estimating the predictive power to discriminate between stable and progressive patients. Protein concentrations for the markers IL1RN and LIFR were elevated in the group of progressive disease patients by folds of 1.29 and 1.16 on average yielding AUC values of 0.632 and 0.634 respectively.

Example 9 - Determination of the diagnostic potential via ELISA on protein level

The diagnostic potential of the marker candidates was determined comparing protein concentrations of markers in plasma of diseased patients (stable and progressive disease) [n=36] and healthy volunteers [n=30]. Protein concentrations for the markers C6, IL1RN, and LIFR were elevated in diseased patients by folds of 1.47, 1.80, and 1.07 on average yielding AUC values of 0.822, 0.706, and 0.565 respectively. The combination of C6 and IL1RN resulted in an increased AUC of 0.834 as compared to an AUC of 0.822 when using C6 alone.

**Claims**

1. A method of determining the risk of progressive renal disease in a patient, by measuring the amount of a marker selected from the group of IL1 RN, ISG15, LIFR, C6, IL32 and any combination thereof, in a sample of said patient.

2. A method according to claim 1, wherein said combination contains at least one of IL1 RN, IL32 and LIFR.

3. A method according to any one of claim 1 or 2, wherein said combination is a combination of two or three markers.

4. The method according to any one of claims 1 to 3, wherein the disease is indicated if the amount of said marker is increased at least 1.1 times the reference value of subjects not suffering from the progressive disease.

5. The method according to any one of claims 1 to 4, wherein the sample is a tissue, blood, serum, plasma or urine sample.

6. The method according to any one of claims 1 to 5, wherein said sample is a kidney biopsy sample.

7. The method according to any one of claims 1 to 6, wherein the expression of said marker is determined.

8. The method according to any one of claims 1 to 7, wherein nucleic acid and/or protein expression of said marker is determined.

9. The method according to any one of claims 1 to 8, wherein said parameter is determined by microarray hybridization with specific probes or by PCR.

10. The method according to any one of claims 1 to 9, wherein a further senescence parameter is measured.

11. The method according to any one of claims 1 to 9, wherein the renal disease is selected from IgA nephropathy, non IgA mesangioproliferative glomerulonephritis, membranoproliferative glomerulonephritis, any postinfectious glomerulonephritis, focal-segmental glomerulosclerosis, minimal change disease, membranous nephropathy, lupus nephritis of any kind, vasculitides with renal involvement of any kind, any other systemic disease leading to renal disease including but not being limited to diabetes mellitus, hypertension or amyloidosis, any hereditary renal disease, any interstitial nephritis and renal transplant failure.

12. Use of a method according to any one of claims 1 to 11, for the prognosis of kidney failure.

13. Use of a method according to any one of claims 1 to 11, for the diagnosis of renal disease in a patient at risk of disease progression.

14. Use of a set of reagents to determine two to five markers selected from the group consisting of IL1 RN, ISG15, LIFR, C6 and IL32 for determining the risk of progressive renal disease in a patient, wherein said reagents are selected from the group of antibodies, antibody fragments and nucleotide sequence specific oligonucleotides.

**15.** Use according to any one of claim 14, wherein said reagents are labelled.

**Patentansprüche**

**1.** Verfahren zum Bestimmen des Risikos einer fortschreitenden Nierenerkrankung bei einem Patienten, indem in einer Probe des Patienten die Menge eines Markers, der aus der Gruppe von IL1 RN, ISG15, LIFR, C6, IL32 und einer Kombination davon ausgewählt ist, gemessen wird.

**2.** Verfahren nach Anspruch 1, wobei die Kombination mindestens IL1RN, IL32 und LIFR enthält.

**3.** Verfahren nach einem der Ansprüche 1 oder 2, wobei die Kombination eine Kombination von zwei oder drei Markern ist.

**4.** Verfahren nach einem der Ansprüche 1 bis 3, wobei die Krankheit angezeigt ist, wenn die Menge des Markers auf mindestens das 1,1fache des Referenzwertes von Personen, die nicht an der fortschreitenden Nierenerkrankung leiden, erhöht ist.

**5.** Verfahren nach einem der Ansprüche 1 bis 4, wobei die Probe Gewebe, Blut, Serum, Plasma oder eine Urinprobe ist.

**6.** Verfahren nach einem der Ansprüche 1 bis 5, wobei die Probe eine Nierenbiopsieprobe ist.

**7.** Verfahren nach einem der Ansprüche 1 bis 6, wobei die Expression des Markers bestimmt wird.

**8.** Verfahren nach einem der Ansprüche 1 bis 7, wobei die Nukleinsäure und/oder die Proteinexpression des Markers bestimmt werden.

**9.** Verfahren nach einem der Ansprüche 1 bis 8, wobei der Parameter durch eine Microarray-Hybridisierung (Hybridisierung mittels DNA-/Genchip) mit spezifischen Sonden oder durch PCR bestimmt wird.

**10.** Verfahren nach einem der Ansprüche 1 bis 9, wobei ein weiterer Altersparameter gemessen wird.

**11.** Verfahren nach einem der Ansprüche 1 bis 9, wobei die Nierenerkrankung aus einer IgA-Nephropathie, einer nicht mesangioproliferativen Glomerulonephritis, einer membranoproliferativen Glomerulonephritis, irgendeiner postinfektiösen bzw. infektionsassoziierten Glomerulonephritis, einer fokal segmentalen Glomerulosklerose, einer Minimal-Change-Glomerulonephritis, einer membranösen Nephropathie, irgendeiner Art von Lupus-Nephritis, irgendeiner Art von Gefäßentzündungen mit Einbeziehung der Niere, irgendeiner anderen systemischen Erkrankung ausgewählt ist, die zu einer Nierenerkrankung führt einschließlich, aber nicht darauf begrenzt, Diabetes mellitus, Bluthochdruck oder Amyloidose, jede erbliche Nierenerkrankung, jede interstitielle Nephritis und ein Nierentransplantationsversagen.

**12.** Verwendung eines Verfahrens nach einem der Ansprüche 1 bis 11 für die Prognose eines Nierenversagens.

**13.** Verwendung eines Verfahrens nach einem der Ansprüche 1 bis 11 für die Diagnose einer Nierenerkrankung bei einem Patienten für das Risiko einer fortschreitenden Erkrankung.

**14.** Verwendung eines Sets von Reagenzien, um zwei bis fünf Marker zu bestimmen, die aus der Gruppe, die aus IL1 RN, ISG15, LIFR, C6 und IL32 besteht, ausgewählt sind, um das Risiko einer fortschreitenden Nierenerkrankung bei einem Patienten zu bestimmen, wobei die Reagenzien aus der Gruppe ausgewählt sind, die aus Antikörpern, Antikörperfragmenten und Nukleotidsequenzen spezifischer Oligonukleotide besteht.

**15.** Verwendung nach einem der Ansprüche 1 bis 14, wobei die Reagenzien bezeichnet sind.

**Revendications**

**1.** Procédé de détermination du risque de maladie rénale progressive dans l'organisme d'un patient, en mesurant la quantité d'un marqueur choisi dans le groupe des ILRN, ISG15, LIFR, C6, IL32 et de toute combinaison de ceux-ci, dans un échantillon dudit patient.

**2.** Procédé selon la revendication 1, dans lequel ladite combinaison contient au moins un élément parmi les IL1RN, IL32 et LIFR.

**3.** Procédé selon l'une quelconque des revendications 1 ou 2, dans lequel ladite combinaison est une combinaison de deux ou trois marqueurs.

**4.** Procédé selon l'une quelconque des revendications 1 à 3, dans lequel la maladie est diagnostiquée si la quantité dudit marqueur est augmentée d'au moins 1,1 fois la valeur de référence des patients ne souffrant pas de la maladie progressive.

**5.** Procédé selon l'une quelconque des revendications de 1 à 4, dans lequel l'échantillon est un tissu, du sang, du sérum, du plasma ou un échantillon d'urine.

**6.** Procédé selon l'une quelconque des revendications 1 à 5, dans lequel ledit échantillon est un échantillon provenant de la biopsie du rein.

**7.** Procédé selon l'une quelconque des revendications 1 à 6, dans lequel l'expression dudit marqueur est déterminée.

**8.** Procédé selon l'une quelconque des revendications 1 à 7, dans lequel l'expression de l'acide nucléique et/ou l'expression de la protéine dudit marqueur est déterminée.

**9.** Procédé selon l'une quelconque des revendications 1 à 8, dans lequel ledit paramètre est déterminé par hybridation micro-réseau avec des sondes spécifiques ou par PCR.

**10.** Procédé selon l'une quelconque des réclamations 1 à 9, dans lequel un paramètre supplémentaire de la sénescence est mesuré.

**11.** Procédé selon l'une quelconque des revendications 1 à 9, dans lequel la maladie rénale est choisie parmi la néphropathie IgA, la glomérulonéphrite mésangioproliférative non à IgA, la glomérulonéphrite membranoproliférative, toute glomérulonéphrite post-infectieuse, la glomérulosclérose segmentaire focale, la maladie à changement minimal, la néphropathie membraneuse, la néphropathie lupique de tout type, les vascularites avec atteinte rénale de tout type, toute autre maladie systémique conduisant à une maladie rénale y compris, sans restriction, le diabète sucré, l'hypertension ou l'amylose, toute maladie rénale héréditaire, toute néphrite interstitielle et l'échec d'une transplantation rénale.

**12.** Utilisation d'un procédé selon l'une quelconque des revendications 1 à 11, pour le pronostic d'une insuffisance rénale.

**13.** Utilisation d'un procédé selon l'une quelconque des revendications 1 à 11, pour le pronostic d'une maladie rénale chez un patient à risque de progression de la maladie.

**14.** Utilisation d'une série de réactifs pour déterminer deux des cinq marqueurs choisis parmi le groupe constitué par IL1 RN, ISG15, LIFR, C6 et IL32 pour la détermination du risque de maladie rénale progressive chez un patient, dans lequel lesdits réactifs sont choisis dans le groupe des anticorps, des fragments d'anticorps et des oligonucléotides spécifiques de la séquence de nucléotides.

**15.** Utilisation selon l'une quelconque de la revendication 14, dans lequel lesdits réactifs sont marqués.

Figure 1:

Figure 2:

Figure 3:

Figure 4:

Figure 5:

LIFR (clone AI820550)

*arrays: ratio to reference RNA* vs. stable / progressive

LIFR (clone AI820550)

*real-time PCR: ratio to housekeeper*; stable / progressive — ratio to GAPDH; stable / progressive — ratio to PPIA

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007028636 A


**Non-patent literature cited in the description**

- **RUDNICKI et al.** *Nephron Exp Nephrol,* 2004, vol. 97, e86-e95
- **RUDNICKI et al.** *Kidney International,* 2007, vol. 71, 325-335
- **RUDNICKI et al.** *Kidney Int,* 2007, vol. 71, 325-35
- **RUDNICKI et al.** *Nephon Exp Nephrol,* 2004, vol. 97, e86-95
- **WÜRZNER et al.** *Clin. Exp. Immunol.,* 1991, vol. 83, 430-437